(19) Europäisches Patentamt
European Patent Office
Office européen des brevets

(11) Publication number: **0 469 690 B1**

(12) **EUROPEAN PATENT SPECIFICATION**

(45) Date of publication of patent specification: **01.02.95**

(51) Int. Cl.$^6$: **A01N 51/00**

(21) Application number: **91202673.9**

(22) Date of filing: **10.08.88**

(60) Publication number of the earlier application in accordance with Art.76 EPC: **0 306 160**

(54) **Molluscicides.**

(30) Priority: **11.08.87 GB 8719006**

(43) Date of publication of application: **05.02.92 Bulletin 92/06**

(45) Publication of the grant of the patent: **01.02.95 Bulletin 95/05**

(84) Designated Contracting States: **AT BE CH DE ES FR GB GR IT LI LU NL SE**

(56) References cited:
**FR-A- 1 188 542**

**CHEMICAL ABSTRACTS, vol. 101, no. 19, 5th November 1984, page 233, abstract no.165584k, Columbus, Ohio, US; & JP-A-59 122 405 (MOON-STAR CHEMICAL CORP.) 14-07-1984**

**CHEMICAL ABSTRACTS, vol. 105, no. 5, 4th August 1986, page 228, abstract no.37193c, Columbus, Columbus, Ohio, US; M. VINCENT et al.: "Effects of two metalsalts (iron(III) chloride, copper(II) chloride) on predation and oviposition by**

(73) Proprietor: **BRITISH TECHNOLOGY GROUP LIMITED
101 Newington Causeway
London SE1 6BU (GB)**

(72) Inventor: **Henderson, Ian Findlay
30 Barlings Road
Harpenden,
Hertfordshire AL5 2AP (GB)**
Inventor: **Bullock, Joseph Ian
9 Vicarage Gate,
Onslow Village
Guildford,
Surrey GU2 5RJ (GB)**
Inventor: **Briggs, Geoffrey Gower
46 Granby Avenue
Harpenden,
Hertfordshire AL5 5OR (GB)**
Inventor: **Larkworthy, Leslie Foot
218 Pampisford Road
South Croydon,
Surrey CR2 6DB (GB)**

**EP 0 469 690 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Zonitoides nitidus Mueller (Mollusca, Gastropoda, pulmonata)", & ANN. RECH.VET. 1986, 17(1), 21-8

(74) Representative: **Wallace, Sheila Jane et al**
**Patents Department**
**British Technology Group Limited**
**101 Newington Causeway**
**GB-London SE1 6BU (GB)**

## Description

This invention relates to molluscicides and in particular to slug and snail poisons.

Chemical Abstracts, vol. 101, No. 19 November 5, 1984, p233 ref No. 165584k, an abstract of JP-A-59 122405 (Moon Star Chemical Corp.) describes the use of compositions containing metal salts, to control gastropods.

Chemical Abstracts, vol. 105, No. 5 August 4, 1986 p228 ref No. 37193c, an abstract of Ann Rech. Vet. 1986, 17(1), 21-8 discloses the molluscicidal effect of iron and cuprous compounds.

It has now been found that certain iron chelates are effective molluscicides.

Accordingly the present invention comprises the use as a molluscicide of a chelate of iron (III) with a ligand of formula II

II: 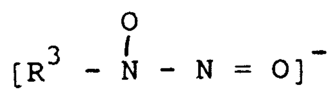

in which formula:

$R^3$ represents:

$C_1$-$C_6$ alkyl, provided that when the alkyl group contains more than four carbon atoms the group is a branched chain alkyl group.

In general, chelates comprising three bidentate ligands, which may be identical or different, though which usually are identical, are preferred and especially those which lose one ligand molecule or ion to form a bis cation.

The ligands of formulae II are readily derived respectively from compounds of formula IIA: $R^3NOH.N=O$, by loss of a proton.

It is preferred that $R^3$ contains no more than four carbon atoms, $R^3$ is preferably methyl, ethyl or n-propyl and the chelate is preferably a trischelate of $Fe^{III}$ comprising identical ligands.

The following compounds of formula IIA are of particular interest:

IIA: $CH_3NOH.N=O$
$CH_3CH_2NOH.N=O$
$CH_3CH_2CH_2NOH.N=O$
$(CH_3)_2CHNOH.N=O$
$CH_3(CH_2)_2CH_2NOH.N=O$
$CH_3CH_2CH(CH_3)NOH.N=O$
$(CH_3)_2CHCH_2NOH.N=O$

The ligands and chelates hereinbefore described may be synthesised by modification of well established routes. Synthesis of complexes comprising the ligand II may be accomplished through the following route:-

$Mg + R^3X \rightarrow R^3MgX$

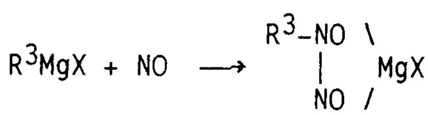

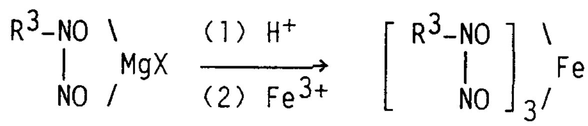


In the foregoing formulae X represents halogen e.g. bromine or iodine.

The present chelates of the present invention may be utilised by formulation in mollusc baits.

Accordingly the present invention further includes within its scope a poison bait for molluscs comprising a molluscicidal chelate as hereinbefore described and a carrier therefor.

The poison bait may comprise, in addition to one or more of the present molluscicidal chelates, other components, for example other molluscicides. Such components may confer additional advantages on the poison bait by, for example, synergising with the present chelates.

The present invention yet further includes with its scope a method of killing a mollusc in which the mollusc or an environment inhabited by the mollusc is treated with a molluscicidal chelate as hereinbefore described.

The present chelates are of particular interest for the control of slugs because the poisons at present used, such as metaldehyde and methiocarb suffer from a disadvantage in that a proportion of the slugs in a treated population, though at first immobilised eventually recover. Environmental pollution as a result of using the chelates is, furthermore, negligible.

The present chelates also offer the advantage that they do not significantly affect ground beetles such as Carabidae beetles, which are attacked by other molluscicides e.g. methiocarb.

Suitable baits usually comprise a mollusc food such as a cereal e.g. wheatmeal, comminuted cuttle fish, starch or gelatin, which may also serve as a carrier. A mollusc phagostimulant such as a sugar e.g. sucrose, or molasses is usually included. Non nutrient carriers of interest include non nutrient polymeric materials, pumice, carbon and materials useful as carriers for insecticides. The bait usually comprises a binder, which is suitably waterproof, such as paraffin wax or casein and may advantageously comprise a bird repellent, for example a blue colourant. In order to inhibit deterioration of the bait a fungistat may be included.

Typically the bait contains at least 3% by weight and no more than 16% by weight of molluscicide. The preferred concentration in the bait is generally 10-13% by weight.

The treatment of both terrestrial and aquatic molluscs in accordance with the present invention is envisaged, the species Deroceras reticulatum, Arion hortensis, Milax budapestensis, Cepaea hortensis, Helix aspersa and Achatina spp being of particular interest as targets.

The invention is illustrated by the following Examples:

Examples 1-8

Bioassay Method

Test compounds were incorporated into a standard wheat-flour-based edible carrier containing 10% paraffin wax binder and 2.5% sucrose phagostimulant, in increasing concentrations 0, 1, 4, 7, 10, 13 and 16% a.i.

Groups of ten field-collected D. reticulatum in the weight range 400-600 mg were confined with 0.5 g DM test bait at each concentration for a period of 4 days under a regime of 12 h dark @ 5 °C and 12 light @ 15 °C. Weight of bait eaten and number of slugs killed were recorded.

The results are shown in Table I. Where six groups of ten slugs are offered bait containing 1,4,7,10,13 or 16% a.i., the results have been reduced to two values, (i), the total amount of a.i. ingested (mg) which gives an indication of the palatability of the compound, and (ii), the total number of slugs killed (x/60) which, with (i), gives an indication of its relative toxicity with metaldehyde and methiocarb under field conditions (grass/clover).

TABLE I

Effect of increasing concentrations of iron chelates in a standard bait on feeding and kill of Deroceras reticulatum

| Example | Compound | a.i. (%) | Eaten (%) | Kill (x/60) |
|---|---|---|---|---|
| 1 | Tris (N-nitroso-N-methyl hydroxylaminato) Fe III 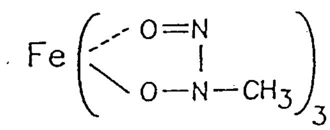 | | 100<br>17<br>11<br>4<br>8<br>5<br>7 | 2<br>5<br>8<br>5<br>5<br>7<br>8 |
| 2 | Tris (N-nitroso-N-methyl hydroxylaminato) Fe III 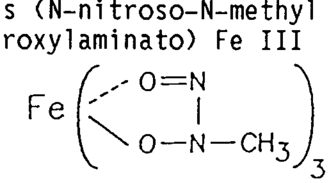 | 17 | 38 | |
| 3 | Tris (N-nitroso-N-ethyl hydroxylaminato) Fe III 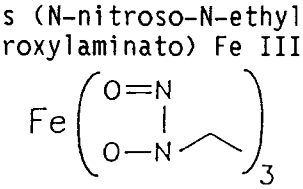 | 6 | 49 | |
| 4 | Tris (N-nitroso-N-n-propyl hydroxylaminato) Fe III 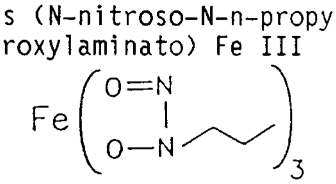 | 50<br>55 | 58<br>58 | |
| 5 | Tris (N-nitroso-N-2-propyl hydroxylaminato) Fe III 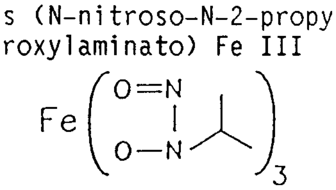 | 3 | 43 | |

| Example | Compound | a.i.ingested (mg/60 slugs) | Slugs dead (x/10) |
|---|---|---|---|
| 6 | Tris (N-nitroso-N-n-butyl hydroxylaminato) Fe III 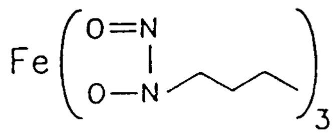 | 53 | 37 |
| 7 | Tris (N-nitroso-N-2-butyl hydroxylaminato) Fe III 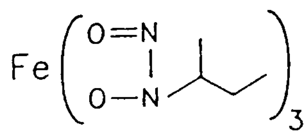 | 54 | 46 |
| 8 | Tris (N-nitroso-N-2-methylpropyl hydroxylaminato) Fe III 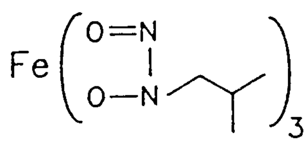 | 160 | 42 |

## Claims

1. The use as a molluscicide of a chelate of iron (III) with a ligand of formula II

II: 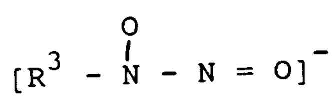

in which formula:
$R^3$ represents:
$C_1$-$C_6$ alkyl , provided that when the alkyl group contains more than four carbon atoms the group is a branched chain alkyl group.

2. A use according to Claim 1, in which the chelate comprises three identical bidentate ligands.

3. A use according to Claim 2, in which $R^3$ contains no more than four carbon atoms.

4. A use according to Claim 3, in which $R^3$ is methyl, ethyl, or n-propyl.

5. A use according to Claim 3, in which the chelate comprises a ligand formed by loss of a proton from one of the following compounds:
$CH_3NOH.N=O$
$CH_3CH_2NOH.N=O$
$CH_3CH_2CH_2NOH.N=O$
$(CH_3)_2CHNOH.N=O$
$CH_3(CH_2)_2CH_2NOH.N=O$

$CH_3CH_2CH(CH_3)NOH.N=O$
$(CH_3)_2CHCH_2NOH.N=O$

6. A use according to Claim 3, in which the chelate is of formula: $Fe^{III}[CH_3NON=O]_3$ or $Fe^{III}[CH_3CH_2NON=O]_3$ or $Fe^{III}[CH_3CH_2CH_2NON=O]_3$.

7. A poison bait for molluscs which comprises a chelate molluscicide as defined in any one of Claims 1 to 6 and a carrier therefor.

8. A poison bait according to Claim 7, which comprises a mollusc food.

9. A poison bait according to Claim 8, in which the mollusc food is a cereal, comminuted cuttle fish, mollases or gelatin.

10. A poison bait according to any of Claims 7 to 9 which comprises a mollusc phagostimulant.

11. A poison bait according to Claim 10, in which the phagostimulant is a sugar or starch.

12. A poison bait according to Claim 7 which comprises a non nutrient carrier.

13. A poison bait according to Claim 12, in which the non nutrient carrier is an artificial polymer, pumice, carbon or a material useful as a carrier for an insecticide.

14. A poison bait according to any of Claims 7 to 13, which comprises a binder.

15. A poison bait according to Claim 14, in which the binder is paraffin wax or casein.

16. A poison bait according to any of Claims 7 to 15, which comprises a bird repellent.

17. A poison bait according to Claim 16, in which the bird repellent is a blue colourant.

18. A poison bait according to any of Claims 7 to 17, which comprises a fungistat.

19. A poison bait according to any of Claims 7 to 18, which comprises at least 3% by weight of a molluscicidal chelate as defined in any one of Claims 1 to 6.

20. A poison bait according to any of Claims 7 to 19, which comprises no more than 16% by weight of a molluscicidal chelate as defined in any one of Claims 1 to 6.

21. A poison bait according to any of Claims 7 to 20, which comprises 10 - 12% by weight of a molluscicidal chelate as defined in any one of Claims 1 to 6.

22. A process for the production of a poison bait for molluscs in which a chelate molluscicide as defined in any one of Claims 1 to 6 is formulated with a carrier therefor.

23. A method of killing a mollusc in which the mollusc or an environment inhabited by the mollusc is treated with a molluscicidal chelate as defined in any one of Claims 1 to 6.

24. A method according to Claim 23, in which an environment inhabited by the mollusc is treated with a poison bait comprising a chelate as defined in any one of Claims 1 to 6.

**Patentansprüche**

**1.** Verwendung eines Eisen(III)-Chelats mit einem Liganden der Formel II

II.

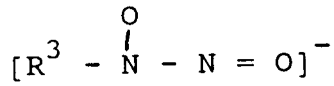

wobei

$R^3$

$C_1$-$C_6$-Alkyl bedeutet, mit der Maßgabe, daß, wenn die Alkylgruppe mehr als vier Kohlenstoffatome enthält, die Gruppe eine verzweigte Alkylgruppe ist,

als Molluskizid bzw. Schneckenbekämpfungsmittel.

**2.** Verwendung nach Anspruch 1, wobei das Chelat drei identische zweizähnige Liganden umfaßt.

**3.** Verwendung nach Anspruch 2, wobei $R^3$ nicht mehr als vier Kohlenstoffatome enthält.

**4.** Verwendung nach Anspruch 3, wobei $R^3$ Methyl, Ethyl oder n-Propyl ist.

**5.** Verwendung nach Anspruch 3, wobei das Chelat einen Liganden umfaßt, der gebildet worden ist durch Verlust eines Protons von einer der folgenden Verbindungen

$CH_3NOH.N=O$

$CH_3CH_2NOH.N=O$

$CH_3CH_2CH_2NOH.N=O$

$(CH_3)_2CHNOH.N=O$

$CH_3(CH_2)_2CH_2NOH.N=O$

$CH_3CH_2CH(CH_3)NOH.N=O$

$(CH_3)_2CHCH_2NOH.N=O$

**6.** Verwendung nach Anspruch 3, wobei das Chelat die Formel

$Fe^{III}[CH_3NON=O]_3$ oder $Fe^{III}[CH_3CH_2NON=O]_3$ oder $Fe^{III}[CH_3CH_2CH_2NON=O]_3$

besitzt.

**7.** Giftköder für Schnecken, umfassend ein Chelat-Molluskizid, nach einem der Ansprüche 1 bis 6, und einen Träger dafür.

**8.** Giftköder nach Anspruch 7, umfassend eine Schneckennahrung.

**9.** Giftköder nach Anspruch 8, bei dem die Schneckennahrung ein Getreide, fein zerteilter Tintenfisch, Melasse oder Gelatine ist.

**10.** Giftköder nach einem der Ansprüche 7 bis 9, umfassend ein Schnecken-Phagostimulans.

**11.** Giftköder nach Anspruch 10, wobei das Phagostimulans ein Zucker oder Stärke ist.

**12.** Giftköder nach Anspruch 7, umfassend einen Nicht-Nahrungsträger.

**13.** Giftköder nach Anspruch 12, wobei der Nicht-Nahrungsträger ein künstliches Polymer, Bimsstein, Kohle oder ein Material, das geeignet ist als Träger für Insektizide, ist.

**14.** Giftköder nach einem der Ansprüche 7 bis 13, umfassend einen Binder.

**15.** Giftköder nach Anspruch 14, wobei der Binder Paraffinwachs oder Kasein ist.

**16.** Giftköder nach einem der Ansprüche 7 bis 15, umfassend ein Abschreckmittel für Vögel.

**17.** Giftköder nach Anspruch 16, wobei das Abschreckmittel für Vögel ein blauer Farbstoff ist.

**18.** Giftköder nach einem der Ansprüche 7 bis 17, umfassend ein Fungistatikum.

**19.** Giftköder nach einem der Ansprüche 7 bis 18, umfassend mindestens 3 Gew.-% eines Molluskizid-Chelats nach einem der Ansprüche 1 bis 6.

**20.** Giftköder nach einem der Ansprüche 7 bis 19, umfassend nicht mehr als 16 Gew.-% eines Molluskizid-Chelats nach einem der Ansprüche 1 bis 6.

**21.** Giftköder nach einem der Ansprüche 7 bis 20, umfassend 10 bis 12 Gew.-% eines Molluskizid-Chelats nach einem der Ansprüche 1 bis 6.

**22.** Verfahren zur Herstellung eines Giftköders für Schnecken, bei dem ein Molluskizid-Chelat nach einem der Ansprüche 1 bis 6 mit einem Träger dafür zubereitet wird.

**23.** Verfahren zum Abtöten einer Schnecke, wobei die Schnecke oder die Umgebung, in der sie sich aufhält, mit einem Molluskizid-Chelat nach einem der Ansprüche 1 bis 6 behandelt wird.

**24.** Verfahren nach Anspruch 23, wobei die Umgebung, in der sich die Schnecke aufhält, mit einem Giftköder behandelt wird, umfassend ein Chelat nach einem der Ansprüche 1 bis 6.

**Revendications**

**1.** Utilisation comme molluscicide d'un chélate de fer(III) avec un ligand de formule II

II :

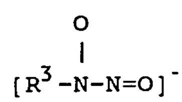

dans laquelle formule :

$R^3$ représente :

un groupe alkyle en $C_1$-$C_6$, sous réserve que, lorsque le groupe alkyle contient plus de quatre atomes de carbone, ce groupe soit un groupe alkyle à chaîne ramifiée.

**2.** Utilisation selon la revendication 1, dans laquelle le chélate comprend trois ligands bidentés identiques.

**3.** Utilisation selon la revendication 2, dans laquelle $R^3$ ne contient pas plus de quatre atomes de carbone.

**4.** Utilisation selon la revendication 3, dans laquelle $R^3$ est un groupe méthyle, éthyle ou n-propyle.

**5.** Utilisation selon la revendication 3, dans laquelle le chélate comprend un ligand formé par perte d'un proton d'un des composés suivants :

$CH_3NOH.N=O$
$CH_3CH_2NOH.N=O$
$CH_3CH_2CH_2NOH.N=O$
$(CH_3)_2CHNOH.N=O$
$CH_3(CH_2)_2CH_2NOH.N=O$
$CH_3CH_2CH(CH_3)NOH.N=O$
$(CH_3)_2CHCH_2NOH.N=O$.

**6.** Utilisation selon la revendication 3, dans laquelle le chélate répond à la formule : $Fe^{III}[CH_3NON=O]_3$ ou $Fe^{III}[CH_3CH_2NON=O]_3$ ou $Fe^{III}[CH_3CH_2CH_2NON=O]_3$.

**7.** Appât toxique pour les mollusques, qui comprend un chélate molluscicide comme défini dans l'une quelconque des revendications 1 à 6 et un support de celui-ci.

**8.** Appât toxique selon la revendication 7, qui comprend un aliment pour mollusques.

**9.** Appât toxique selon la revendication 8, dans lequel l'aliment pour mollusques est une céréale, un broyat de seiche, de la mélasse ou de la gélatine.

**10.** Appât toxique selon l'une quelconque des revendications 7 à 9, qui comprend un appétitif pour mollusques.

**11.** Appât toxique selon la revendication 10, dans lequel l'appétitif est un sucre ou de l'amidon.

**12.** Appât toxique selon la revendication 7, qui comprend un support non nutritif.

**13.** Appât toxique selon la revendication 12, dans lequel le support non nutritif est un polymère artificiel, de la pierre ponce, du charbon ou une matière utile comme support pour un insecticide.

**14.** Appât toxique selon l'une quelconque des revendications 7 à 13, qui comprend un liant.

**15.** Appât toxique selon la revendication 14, dans lequel le liant est de la cire de paraffine ou de la caséine.

**16.** Appât toxique selon l'une quelconque des revendications 7 à 15, qui comprend un répulsif aviaire.

**17.** Appât toxique selon la revendication 16, dans lequel le répulsif aviaire est un colorant bleu.

**18.** Appât toxique selon l'une quelconque des revendications 7 à 17, qui comprend un fongistatique.

**19.** Appât toxique selon l'une quelconque des revendications 7 à 18, qui comprend au moins 3 % en poids d'un chélate molluscicide, comme défini dans l'une quelconque des revendications 1 à 6.

**20.** Appât toxique selon l'une quelconque des revendications 7 à 19, qui ne comprend pas plus de 16 % en poids d'un chélate molluscicide, comme défini dans l'une quelconque des revendications 1 à 6.

**21.** Appât toxique selon l'une quelconque des revendications 7 à 20, qui comprend 10 à 12 % en poids d'un chélate molluscicide comme défini dans l'une quelconque des revendications 1 à 6.

**22.** Procédé pour la production d'un appât toxique pour les mollusques, dans lequel un chélate molluscicide, comme défini dans l'une quelconque des revendications 1 à 6, est formulé avec un de ses supports.

**23.** Procédé pour tuer un mollusque, dans lequel le mollusque ou un environnement habité par le mollusque est traité avec un chélate molluscicide, comme défini dans l'une quelconque des revendications 1 à 6.

**24.** Procédé selon la revendication 23, dans lequel un environnement habité par le mollusque est traité avec un appât toxique comprenant un chélate comme défini dans l'une quelconque des revendications 1 à 6.